# EUROPÄISCHE PATENTSCHRIFT

(11) **EP 0 556 674 B1**
(45) Veröffentlichungstag und Bekanntmachung des Hinweises auf die Patenterteilung: **19.06.1996**
(21) Anmeldenummer: 93101842.8
(22) Anmeldetag: 05.02.1993
(51) Int. Cl.: C07C 317/42, C07C 311/47, A61K 31/155

(54) **3,5-Substituierte Benzoylguanidine, mit antiarrythmischer Wirkung und inhibierender Wirkung auf die Proliferationen von Zellen**
3,5-substituted benzoylguanidines with antiarrhythmic activity and cell proliferation inhibiting activity
Benzoylguanidines 3,5-substitués avec une activité antiarrhythmique et une activité inhibitrice de la prolifération des cellules

(30) Priorität: 15.02.1992 DE 4204575
(43) Veröffentlichungstag der Anmeldung: 25.08.1993
(73) Patentinhaber: HOECHST AKTIENGESELLSCHAFT, D-65926 Frankfurt am Main (DE)
(72) Erfinder: Lang, Hans-Jochen, Dr., W-6238 Hofheim/Ts (DE); Mania, Dieter, Dr., W-6240 Königstein/Ts. (DE); Weichert, Andreas, Dr., W-6000 Frankfurt/M. (DE); Scholz, Wolfgang, Dr., W-6236 Eschborn (DE); Albus, Udo, Dr., W-6364 Florstadt (DE); Englert, Heinrich, Dr., W-6238 Hofheim/Ts. (DE); Lang, Florian, Prof. Dr., W-7778 Markdorf (DE)

(56) Entgegenhaltungen:
- EP-A- 0 416 499
- US-A- 3 780 027
- BIOCHEMICAL AND BIOPHYSICAL RESEARCH COMMUNICATIONS, Bd. 184, Nr. 1, 15. April 1992, Dulluth, MN, US, Seiten 112 - 117 A. SCHMID, ET AL.: 'Na+/H+ exchange in porcine cerebral capillary endothelial cells inhibited by a benzoylguanidine derivative'

## Beschreibung

Die Erfindung betrifft Benzoylguanidine der Formel I 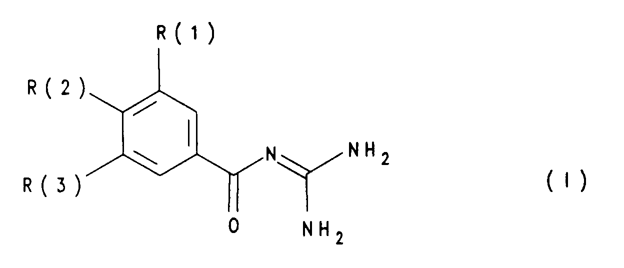
worin bedeuten:
- R(1): R(4)-SOₘ oder R(5)R(6)N-SO₂-, mit
m gleich Null, 1 oder 2,
R(4) und R(5) gleich C₁-C₈-Alkyl, C₃-C₆-Alkenyl, -CₙH₂ₙ-R(7),
n gleich Null, 1, 2, 3 oder 4,
R(7) gleich C₅-C₇-Cycloalkyl, Phenyl, welches unsubstituiert ist oder substituiert mit 1-3 Substituenten aus der Gruppe
F, Cl, CF₃, Methyl, Methoxy oder NR(8)R(9) mit
R(8) und R(9) gleich H oder C₁-C₄-Alkyl,
wobei R(5) auch in der Bedeutung von H steht,
R(6) gleich H oder C₁-C₄-Alkyl,
wobei R(5) und R(6) gemeinsam 4 oder 5 Methylengruppen sein können, von denen eine CH₂-Gruppe durch ein O, S, NH, N-CH₃ oder N-Benzyl ersetzt sein kann,
- R(2): Wasserstoff, F, Cl, Br, (C₁-C₄)-Alkyl-, O-(CH₂)ₘCₚF₂ₚ₊₁, -X-R(10), mit
m gleich Null oder 1,
p gleich 1,2 oder 3,
X gleich O, S, NR(11),
R(10) gleich H, C₁-C₆-Alkyl, C₅-C₇-Cycloalkyl, Cyclohexylmethyl, Cyclopentylmethyl, -CₙH₂ₙ-R(12) mit
n gleich Null, 1, 2, 3 oder 4 und
R(12) gleich Phenyl, das unsubstituiert oder substituiert ist mit 1-3 Substituenten aus der Gruppe
F, Cl, CF₃, Methyl, Methoxy oder NR(8)R(9) mit
R(8) und R(9) gleich H oder C₁-C₄-Alkyl,
R(11) gleich H oder C₁-C₃-Alkyl,
wobei R(10) und R(11) auch gemeinsam 4 oder 5 Methylengruppen sein können und eine CH₂-Gruppe durch O, S, NH, N-CH₃ oder N-Benzyl ersetzt sein kann,
- R(3): wie R(1) definiert, oder C₁-C₆-Alkyl, Nitro, Cyano, Trifluormethyl, F, Cl, Br, J,-X-R(10) mit
X gleich O, S, NR(11),
R(10) gleich H, C₁-C₆-Alkyl, C₅-C₇-Cycloalkyl, Cyclohexylmethyl,
Cyclopentylmethyl, -CₙH₂ₙ-R(12) mit
n gleich von null bis 4 und
R(12) gleich Phenyl, das unsubstituiert ist oder substituiert mit 1 -3 Substituenten aus der Gruppe
F, Cl, CF₃, Methyl, Methoxy oder NR(8)R(9) mit R(8) und
R(9) gleich H oder C₁-C₄-Alkyl,
R(11) gleich H oder C₁-C₃-Alkyl,
wobei R(10) und R(11) auch gemeinsam 4 oder 5 Methylengruppen sein können und eine CH₂-Gruppe durch O, S, NH, N-CH₃ oder N-Benzyl ersetzt sein kann,
sowie deren pharmazeutisch verträgliche Salze,
wobei jedoch Verbindungen ausgenommen sind, in denen gleichzeitig bedeuten:
- R(1): R(4)-SOₘmit m = Null bis 2 oder R(5)R(6) NSO₂-,
- R(2): Halogen, (C₁-C₄)-Alkyl, und
- R(3): -NR(10)R(11).

Bevorzugt sind Verbindungen der Formel I, in denen bedeuten:
- R(1): R(4)-SOₘ oder R(5)R(6)N-SO₂-, mit
m gleich Null, 1 oder 2,
R(4) gleich Methyl oder -CₙH₂ₙ-R(7),
n gleich Null oder 1
R(7) gleich Phenyl, welches unsubstituiert ist oder substituiert mit 1-3 Substituenten aus der Gruppe
Cl, CF₃, Methyl oder Methoxy,
R(5) gleich H, C₁-C₆-Alkyl, Allyl, -CₙH₂ₙ-R(7),
n gleich Null, 1, 2 oder 3,
R(7) gleich Phenyl, welches unsubstituiert ist oder substituiert mit 1-3 Substituenten aus der Gruppe
F, Cl, CF₃, Methyl, Methoxy oder NR(8)R(9) mit
R(8) und R(9) gleich H oder Methyl,
R(6) gleich H oder Methyl,
wobei R(5) und R(6) gemeinsam 4 oder 5 Methylengruppen sein können, von denen eine CH₂-Gruppe durch ein O, S, N-CH₃ oder N-Benzyl ersetzt sein kann,
- R(2): Wasserstoff, O-CH₂CF₃ oder -X-R(10), mit
X gleich O, S, NR(11),
R(10) gleich H, C₁-C₆-Alkyl, -CₙH₂ₙ-R(12) mit
n für X in der Bedeutung von Sauerstoff oder Schwefel gleich Null, und für X in der Bedeutung von NR(12) gleich Null oder 1, und
R(12) gleich Phenyl, das unsubstituiert oder substituiert ist mit 1-3 Substituenten aus der Gruppe
F, Cl, CF₃, Methyl,
R(11) gleich H, (C₁-C₃)-Alkyl,
wobei R(10) und R(11) auch gemeinsam 4 oder 5 Methylengruppen sein können und eine CH₂-Gruppe durch O, S, N-CH₃ oder N-Benzyl ersetzt sein kann,
- R(3): Methyl, Nitro, Cyano, Trifluormethyl, F, Cl, -X-R(10) mit
X gleich O, S, NR(11),
R(10) gleich H, C₁-C₆-Alkyl, -CₙH₂ₙ-R(12) mit
n gleich null oder 1
R(12) gleich Phenyl, das unsubstituiert ist oder substituiert mit 1 - 3 Substituenten aus der Gruppe Cl, CF₃, Methyl, Methoxy,
R(11) gleich H oder (C₁-C₃)-Alkyl
wobei R(10) und R(11) auch gemeinsam 4 oder 5 Methylengruppen sein können,
sowie deren pharmazeutisch verträgliche Salze.

Besonders bevorzugt sind Verbindungen der Formel I, in denen bedeuten:
- R(1): (R4)-SO₂- oder R(5)R(6)N-SO₂- mit
- R(4): gleich Methyl,
- R(5) und R(6): gleich Wasserstoff,
- R(2): Wasserstoff, -O-CH₂-CF₃ oder -X-R(10) mit
X gleich Sauerstoff, N-R(11),
R(10) gleich H, C₁-C₆-Alkyl, -CₙH₂ₙ-R(12) mit
n = Null oder 1
R(12) gleich Phenyl, das unsubstituiert oder substituiert ist mit einem Substituenten aus der Gruppe F, Cl oder Methyl,
R(11) gleich H oder C₁-C₃-Alkyl ist,
- R(3): Methyl, F, Cl,
sowie deren pharmakologisch verträgliche Salze.

Ganz besonders bevorzugt sind die Verbindungen 3-Chlor-4-N,N-diethylamino-5-methylsulfonylbenzoyl-guanidin-hydrochlorid, 3,4-Dimethyl-5-sulfamoylbenzoyl-guanidin-hydrochlorid.

Enthält einer der Substituenten R(1) bis R(12) ein Asymmetriezentrum, so gehören sowohl S als auch R konfigurierte Verbindungen zur Erfindung. Die Verbindungen können als optische Isomere, als Diastereomere, als Racemate oder als Gemische derselben vorliegen.

Die bezeichneten Alkylreste können sowohl geradkettig wie verzweigt vorliegen.

Die Erfindung betrifft weiterhin ein Verfahren zur Herstellung der Verbindungen I, dadurch gekennzeichnet, daß man

### Verbindungen der Formel II

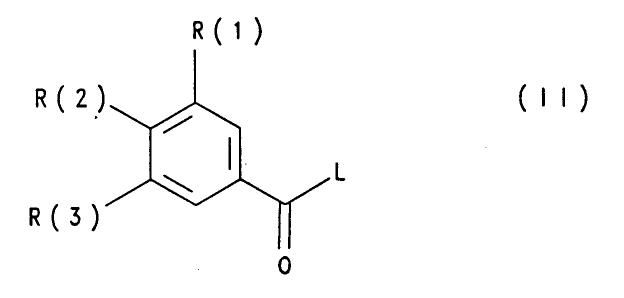

mit Guanidin umsetzt, worin R(1) bis R(3) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L=Cl), die man ihrerseits wiederum in an sich bekannter Weise aus den zugrundeliegenden Carbonsäuren (Formel II, L= OH) beispielsweise mit Thionylchlorid herstellen kann.
Neben den Carbonsäurechloriden der Formel II (L=Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L=OCH₃ durch Behandeln mit gasförmigem HCl in Methanol, die Imidazolide der Formel II durch Behandeln mit Carbonyldiimidazol (L= 1-Imidazolyl, Staab, Angew. Chem. Int. Ed. Engl. 1, 351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluroniumtetrafluoborat ("TOTU")(Weiss und Krommer, Chemiker Zeitung 98, 817 (1974)). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L=OMe) mit Guanidin Methanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L=Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäurederivate der Formel II sind bekannt und in der Literatur beschrieben. Die unbekannten Verbindungen der Formel II können nach literaturbekannten Methoden hergestellt werden.

Carbonsäuren oder deren Ester der Formel II (z.B. L = -OH oder -O-Methyl) mit R(2) in der Bedeutung von Halogen oder R(3) in der Bedeutung von Nitro können als vielseitige Ausgangsverbindungen für weitere Carbonsäuren der Formel II dienen, wobei das Halogen in der Position R(2) sehr bequem in bekannter Weise gegen zahlreiche nucleophile Reagenzien, wie Mercaptane R(10)-SH, Phenole R(10)-OH oder primäre oder sekundäre Aminen R(10)R(11)NH unter Bildung weiterer Benzoesäurederivate II mit L = -OH bzw. -O-Methyl ausgetauscht werden kann oder Nitro nach reduktiver Umwandlung in NH₂ in zahlreichen (Alkylierungen, Acylierungen, Diazotierungen mit nachfolgenden Sandmeyer-, Ullmann- Meerwein-usw. Reaktionen) in weitere Benzoesäurederivate II mit L = -OH bzw. -O-Methyl übergeführt werden können.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 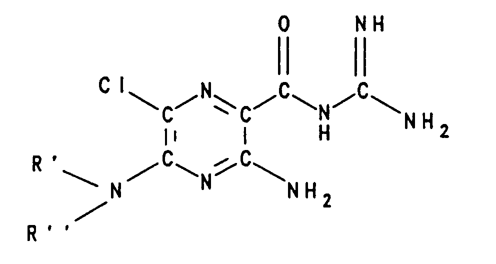
Amilorid: R',R'' = H
Dimethylamilorid: R',R''= CH₃
Ethylisopropylamilorid: R' = C₂H₅, R'' = CH(CH₃)₂
Darüberhinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.
Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelostes Kammerflimmern durch Amilorid völlig unterdrückt werden konnte. Noch potenter als Amilorid war in diesem Modell das oben erwähnte Amiloridderivat Ethylisopropylamilorid.

In der US Patentschrift 5 091 394 sind Benzoylguanidine beschrieben, die in der dem Rest R(3) entsprechenden Stellung ein Wasserstoffatom tragen.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen. Die Verbindungen sind infolge ihrer pharmakologischen Eigenschaften als antiarrhytmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na+ /H+ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na+/H+ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Ent-schäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg bis 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Allgemeine Vorschrift zur Herstellung von Benzoyl-guanidinen (I) aus Benzoesäuren (II, L=OH)

0,01 M des Benzoesäurederivates der Formel II löst bzw. suspendiert man in 60 ml wasserfreiem Tetrahydrofuran (THF) und versetzt sodann mit 1,78 g (0,011 M) Carbonyldiimidazol. Nach dem Rühren über 2 Stunden bei Zimmertemperatur werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6-7 und filtriert das entsprechende Benzoylguanidin (Formel I) ab. Die so erhaltenen Benzoylguanidine können durch Behandeln mit wäßriger oder methanolischer Salzsäure oder anderen pharmakologisch verträglichen Säuren in die entsprechenden Salze übergeführt werden.

### Beispiel 1:

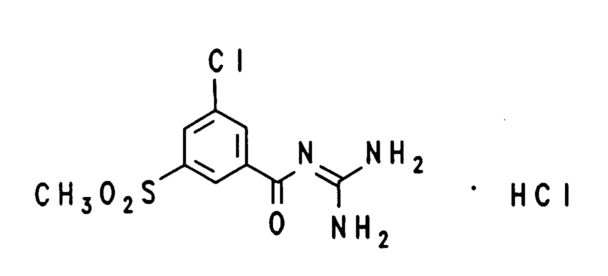

3-Chlor-5-methylsulfonylbenzoyl-guanidin-hydrochlorid:
Farblose Kristalle, Schmp. 230°C.
Syntheseweg:
a) Reduktion von 3-Chlor-5-chlorsulfonylbenzoesäure zu 3-Chlor-5-carboxy-benzolsulfinsäure mit Natriumbisulfit in Wasser bei 10-15°C und konstantem pH 8 - 9 (NaOH, Glaselektrode), acidifizieren mit HCl und abfiltrieren des weißen Niederschlages.
b) 3-Chlor-5-carboxy-benzolsulfinsäure-dinatriumsalz aus a) mit 2 Äquivalenten NaOH in Wasser, eindampfen, suspendieren in Aceton und Kristalle abfiltrieren; Weiße Kristalle, Schmp. > 300 °C.
c) 3-Chlor-5-methylsulfonylbenzoesäure-methylester aus b) mit 1 Äquivalent Methyljodid in DMF bei 80°C/8 Stunden umsetzen, Lösungsmittel abdestillieren, Säulenchromatografie an Kieselgel und Essigester/Toluol-Gemisch (1:3). Farblose Kristalle, Schmp. 75°C.
d) 3-Chlor-5-methylsulfonylbenzoesäure aus c) durch alkalische Hydrolyse und Ansäuern mit HCl. Weißes kristallines Pulver, Schmp. 214°C.
e) 3-Chlor-5-methylsulfonylbenzoyl-guanidin-hydrochlorid aus d) nach Allgemeiner Vorschrift (siehe oben). Farblose Kristalle, Schmp. 230°C.

### Beispiel 2:

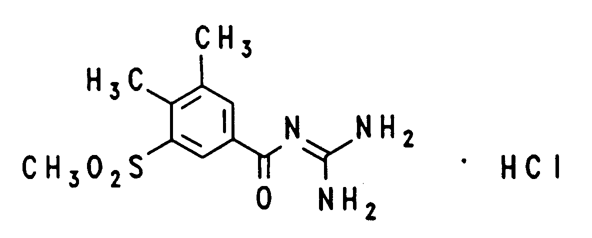

3,4-Dimethyl-5-methylsulfonylbenzoyl-guanidin-hydrochlorid; farblose Kristalle, Schmp. 288°C.
Syntheseweg:
a) 2,3-Dimethyl-5-carboxybenzolsulfinsäure aus 5-Chlorsulfonyl-3,4-dimethylbenzoyl-benzoesäure durch Reduktion mit Natriumbisulfit analog Beispiel 1,a), weiße Kristalle, Schmp. 213°C.
b) 2,3-Dimethyl-5-carboxybenzolsulfinsäure-dinatriumsalz aus 2,3-Dimethyl-5-carboxybenzolsulfinsäure analog Beispiel 1,b). Farblose Kristalle, Schmp. > 320°C.
c) 3,4-Dimethyl-5-methylsulfonylbenzoesäure-methylester aus 2,3-Dimethyl-5-carboxybenzolsulfinsäure-dinatrumsalz analog Beispiel 1,c). Farblose Kristalle, Schmp. 102°C.
d) 3,4-Dimethyl-5-methylsulfonylbenzoesäure aus 3,4-Dimethyl-5-methylsulfonylbenzoesäure-methylester analog Beispiel 1,d); Farblose Kristalle aus Ethanol, Schmp. 224°C.
e) 3,4-Dimethyl-5-methylsulfonylbenzoyl-guanidin-hydrochlorid nach allgemeiner Vorschrift aus 3,4-Dimethyl-5-methylsulfonylbenzoesäure. Farblose Kristalle, Schmp. 288°C.

### Beispiel 3:

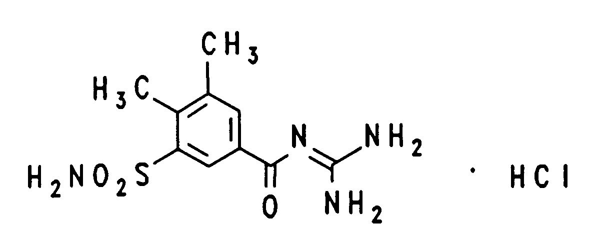

3,4-Dimethyl-5-sulfamoylbenzoyl-guanidin-hydrochlorid nach allgemeiner Vorschrift aus 3,4-Dimethyl-5-sulfamoylbenzoesäure Farblose Kristalle, Schmp. 270°C.

### Beispiel 4

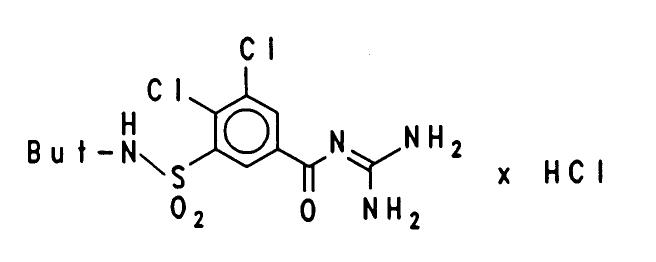

5-(1-Butylsulfamoyl)-3,4-dichlorbenzoylguanidin-hydrochlorid wird erhalten durch Umsetzung von 3,4-Dichlorbenzoesäure in Chlorsulfonsäure bei 140-160°C unter Erhalt von 3,4-Dichlor-5-chlorsulfonylbenzoesäure (Schmp. 203°C), Reaktion mit N-Butylamin zu 5-(1-Butylsulfamoyl)-3,4-dichlorbenzoesäure (Schmp. 160-165°C) und deren nachfolgender Aktivierung mit Carbonyldiimidazol, Umsetzung mit Guanidin und Behandlung mit HCl zu 5-(1-Butylsulfamoyl)-3,4-dichlorbenzoylguanidin-hydrochlorid, Schmp. 140-145°C.

### Beispiel 5

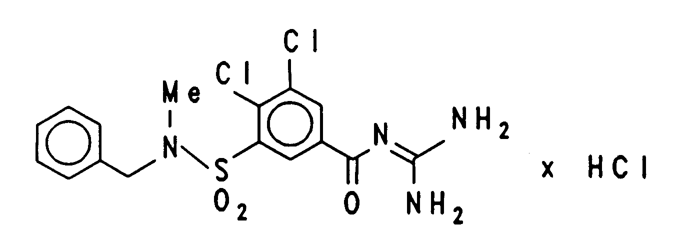

5-N-Benzyl-N-methylsulfamoyl-3,4-dichlorbenzoyl-guanidin-hydrochlorid wird erhalten durch Umsetzung von 3,4-Dichlor-5-chlorsulfonyl-benzoesäure mit N-Methylbenzylamin zu 5-N-Benzyl-N-methylsulfamoyl-3,4-dichlorbenzoesäure (Schmp. 155-160°) und nachfolgender Reaktion gemäß allgemeiner Vorschrift (siehe oben):
5-N-Benzyl-N-methylsulfamoyl-3,4-dichlorbenzoyl-guanidin-hydrochlorid. Farblose Kristalle, Schmp. 185-190°C.

### Beispiel 6

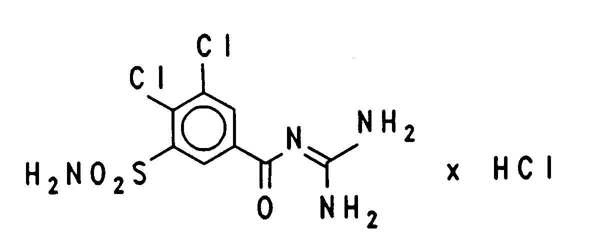

3,4-Dichlor-5-sulfamoylbenzoyl-guanidin-hydrochlorid
(Farblose kristalline Substanz, Schmp. 234-36°C) wird erhalten aus 3,4-Dichlor-5-sulfamoylbenzoesäure gemäß allgemeiner Vorschrift (siehe oben).

### Beispiel 7

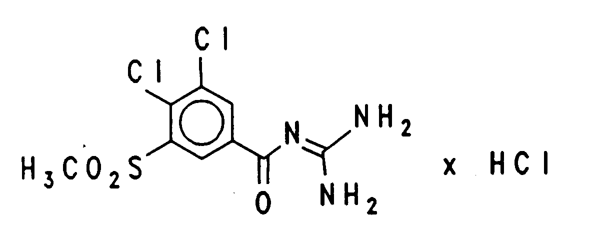

3,4-Dichlor-5-methylsulfonylbenzoyl-guanidin-hydrochlorid
Farblose Kristalle, Schmp. 236-240°C, wird erhalten ausgehend von 3,4-Dichlor-5-chlorsulfonyl-benzoesäure durch Sulfitreduktion zu 2,3-Dichlor-5-carboxyl-benzolsulfinsäure-dinatriumsalz (Schmp. > 300°C), Umsetzung mit überschüssigem Methyliodid zu 3,4-Dichlor-5-methylsulfonylbenzoesäure-methylester, Hydrolyse zur 3,4-Dichlor-5-methylsulfonylbenzoesäure (196-199°C) und anschließender Umsetzung nach allgemeiner Vorschrift.

### Beispiel 8

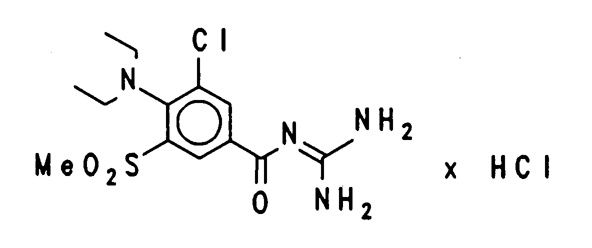

3-Chlor-4-N,N-diethylamino-5-methylsulfonylbenzoyl-guanidin-hydrochlorid Farblose Kristalle, Schmp.: 222-224°C wird erhalten ausgehend von 3,4-Dichlor-5-methylsulfonyl-benzoesäure-methylester durch Reaktion mit Diethylamin im Autoklaven (160°C, 12 Stunden) und anschließende alkalische Hydrolyse zur 3-Chlor-4-N,N-diethylamino-5-methylsulfonyl-benzoesäure (Schmp. 115-117°C) und Umsetzung mit Guanidin nach Vorschrift (siehe oben).

### Beispiel 9

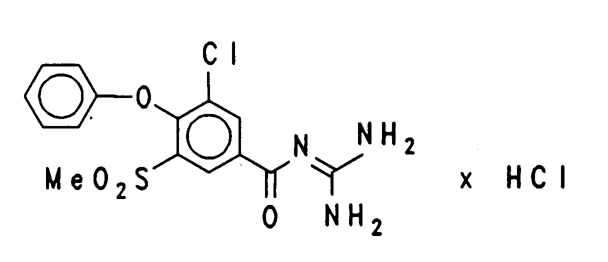

3-Chlor-5-methylsulfonyl-4-phenoxybenzoyl-guanidin-hydrochlorid;
Farblose Kristalle, Schmp. 271-274°C, wird erhalten ausgehend von 3,4-Dichlor-5-methylsulfonyl-benzoesäure-methylester durch Reaktion mit Phenol und Kaliumcarbonat in DMF (80°C, 6,5 Stunden) und anschließender alkalischer Hydrolyse zur 3-Chlor-5-methylsulfonyl-4-phenoxy-benzoesäure (Schmp. 210-212°C) und Umsetzung mit Guanidin nach Vorschrift (siehe oben).

### Beispiel 10

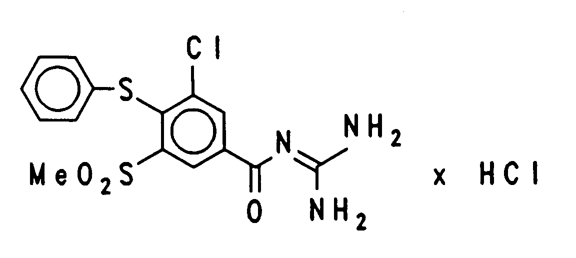

3-Chlor-5-methylsulfonyl-4-phenylthiobenzoyl-guanidin-hydrochlorid;
Farblose Kristalle,Schmp. 228-230°C, wird erhalten ausgehend von 3,4-Dichlor-5-methylsulfonyl-benzoesäure-methylester durch Reaktion mit Thiophenol und Kaliumcarbonat in DMF (120°C, 6,5 Stunden) und anschließender alkalischer Hydrolyse zur 3-Chlor-5-methylsulfonyl-4-phenylthio-benzoesäure (Schmp. 231-233°C) und Umsetzung mit Guanidin nach Vorschrift (siehe oben).

### Beispiel 11

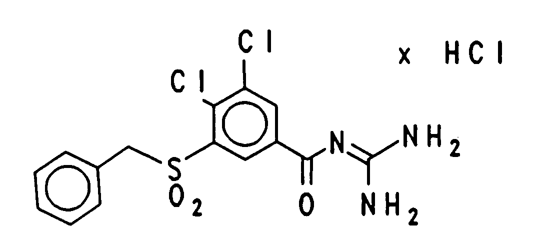

3-Benzylsulfonyl-4,5-dichlorbenzoyl-guanidin-hydrochlorid;
Farblose Kristalle, Schmp.: 262°C wird erhalten durch Reaktion von 2,3-Dichlor-5-carboxyl-benzolsulfinsäure-dinatriumsalz mit 2 Äquivalenten Benzylchlorid in DMF (80°C, 5 Stunden) und nachfolgender saurer Hydrolyse (20%ige wäßrige HCl, in Eisessig, 3 Stunden Rückfluß) zu 5-Benzylsulfonyl-3,4-dichlor-benzoesäure (Schmp. 183°C) und nachfolgender Umsetzung mit Guanidin nach allgemeiner Vorschrift.

### Beispiel 12

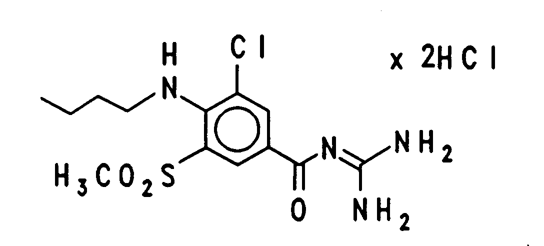

4-(1-Butylamino)-3-chlor-5-methylsulfonylbenzoyl-guanidin-dihydrochlorid, Farblose Kristalle, Schmp.: 200°C wird erhalten ausgehend von 3,4-Dichlor-5-methylsulfonyl-benzoesäure (Schmp. 215-220°C) durch Reaktion mit n-Butylamin im Autoklaven (140°C, 12 Stunden) zu 4-(1-Butylamino)-3-chlor-5-methylsulfonylbenzoesäure (Schmp. 155-160°C) und Umsetzung mit Guanidin nach Vorschrift (siehe oben).

### Beispiel 13

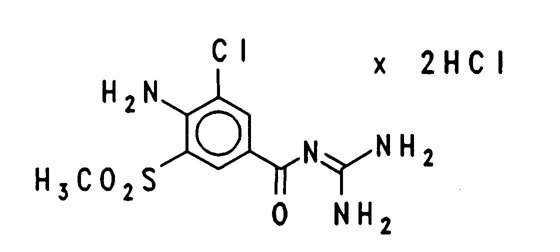

4-Amino-3-chlor-5-methylsulfonylbenzoyl-guanidin-dihydrochlorid,
Farblose Kristalle, Schmp.: 270°C wird erhalten ausgehend von 4-Amino-3-chlor-5-methylsulfonyl-benzoesäure (Schmp. 255-261°C) durch Umsetzung mit Guanidin nach Vorschrift (siehe oben).

### Beispiel 14

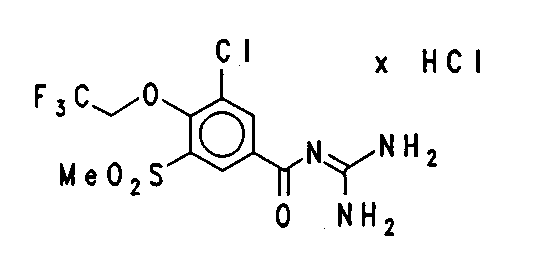

3-Chlor-4-(2,2,2-trifluorethoxy)-5-methylsulfonyl-benzoylguandin-hydrochlorid;
farblose Kristalle, Schmp. 261°C wird erhalten ausgehend von 3,4-Dichlor-5-methylsulfonyl-benzoesäure-methylester durch Reaktion mit 2,2,2-Trifluorethanol und Kaliumcarbonat in DMF (100°C, 6 Stunden) und anschließender alkalischer Hydrolyse zur 3-Chlor-4-(2,2,2-trifluorethoxy)-5-methylsulfonyl-benzoesäure (Schmp. 214-220°C) und Umsetzung mit Guanidin nach Vorschrift (siehe oben).

### Beispiel 15

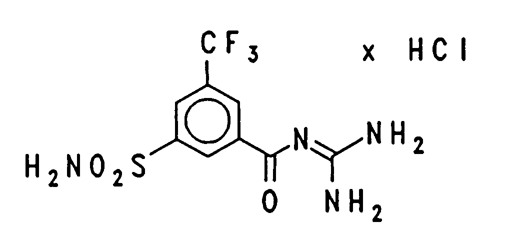

3-Sulfamoyl-5-trifluormethylbenzoyl-guanidin-hydrochlorid;
farblose Kristalle, Schmp. 235-238°C erhält man durch Reaktion von 3-Trifluormethylbenzoesäure durch Nitrierung (100%ige HNO₃ in 20 %igem Oleum, 3 Stunden Raumtemperatur) zur 5-Nitro-3-trifluormethylbenzoesäure (Schmp. 126-129°C).
Katalytische Hydrierung (Platin-IV-oxid in Ethanol bei Raumtemperatur, 1 atm) zur 5-Amino-3-trifluormethylbenzoesäure (Schmp. 133-137°C),
Meerwein-Reaktion zur 5-Chlorsulfonyl-3-trifluormethylbenzoesäure (Schmp. 144-147°C), Umsetzung mit wäßrigem Ammoniak (25 %ig, 14 Stunden Raumtemperatur) zu 5-Sulfamoyl-3-trifluormethylbenzoesäure (Schmp. 235-240°C) und Umsetzung mit Guanidin nach Vorschrift (siehe oben).

### Beispiel 16

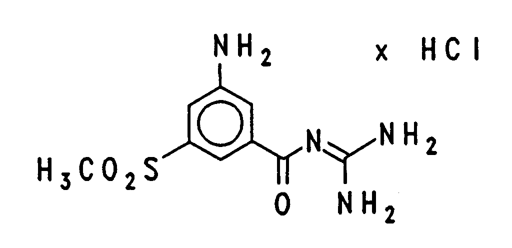

3-Amino-5-methylsulfonyl-benzoylguanidin-dihydrochlorid,
farblose Kristalle, Schmp. 264°C, erhält man durch Reaktion von 3-Methylsulfonylbenzoesäure durch Nitrierung (100%ige HNO₃ in 20%igem Oleum, 3 Stunden Raumtemperatur) zur 5-Nitro-3-methylsulfonylbenzoesäure (Schmp. 218-220°C),
katalytische Hydrierung (Raney-Nickel in Methanol bei Raumtemperatur, 1 atm) zur 5-Amino-3-methylsulfonylbenzoesäure (Schmp. 300-310°C), und Umsetzung mit Guanidin nach Vorschrift (siehe oben).

### Beispiel 17

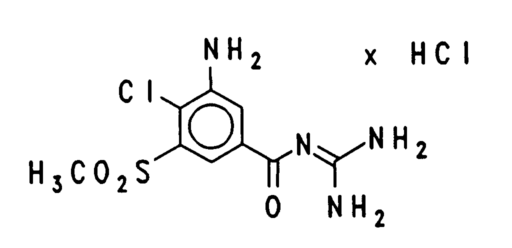

3-Amino-4-chlor-5-methylsulfonylbenzoyl-guanidin-dihydrochlorid,
farblose Kristalle, Schmp. 279-281°C., erhält man durch Reaktion von 4-Chlor-3-methylsulfonylbenzoesäure durch Nitrierung (100 %ige HNO₃ in 20%igen Oleum, 4 Stunden bei 90-100°C) zur 4-Chlor-5-nitro-3-methylsulfonylbenzoesäure (Schmp. 190-194°C),
Reduktion mit Natriumbisulfit (1 Stunde, 100°C in Wasser) zur 5-Amino-4-chlor-3-methylsulfonylbenzoesäure (Schmp. 265-267°C) und Umsetzung mit Guanidin nach Vorschrift (siehe oben).

### Beispiel 18

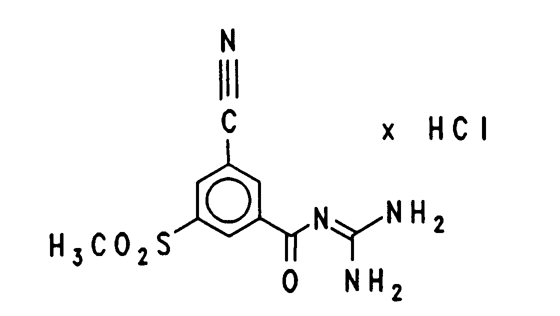

3-Cyano-5-methylsulfonylbenzoyl-guanidin-hydrochlorid,
farblose, schwach gelbe Kristalle, Schmp. 275-278°C (Zers.), erhält man durch Sandmeyer-Reaktion von 3-Amino-5-methylsulfonyl-benzoesäure mit Cu-I-cyanid zu 3-Cyano-5-methylsulfonyl-benzoesäure (Schmp. 226-228) und nachfolgender Umsetzung mit Guanidin nach Vorschrift (siehe oben).

### Beispiel 19

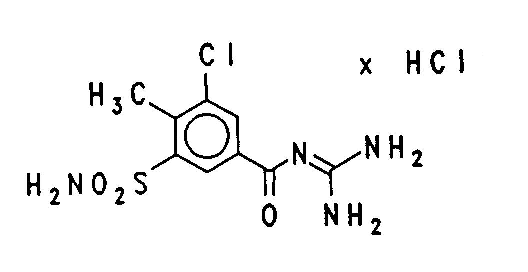

3-Chlor-4-methyl-5-sulfamoylbenzoyl-guanidin-hydrochlorid;
farblose Kristalle,Schmp. 256-259°C, werden aus 3-Chlor-4-methyl-5-sulfamoylbenzoesäure durch Umsetzung mit Guanidin nach Vorschrift (siehe oben) erhalten.

### Beispiel 20

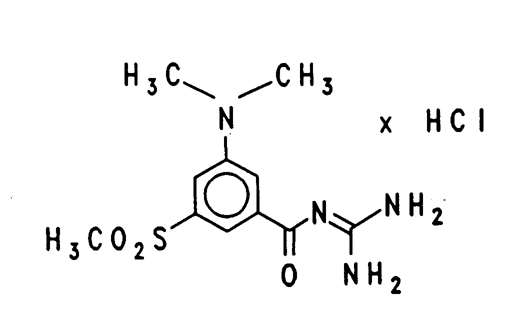

3-Dimethylamino-5-methylsulfonylbenzoyl-guanidin-dihydrochlorid;
farblose Kristalle, Schmp. 278-290°C (Zers.), erhält man durch Umsetzung von 3-Amino-5-methylsulfonylbenzoesäure mit Methyliodid in DMF/Kaliumcarbonat (6 Stunden, 70°C) und anschließender Hydrolyse zur 3-Dimethylamino-5-methylsulfonylbenzoesäure (Schmp. 200-203°C) und nachfolgender Umsetzung mit Guanidin nach Vorschrift (siehe oben).

### Beispiel 21

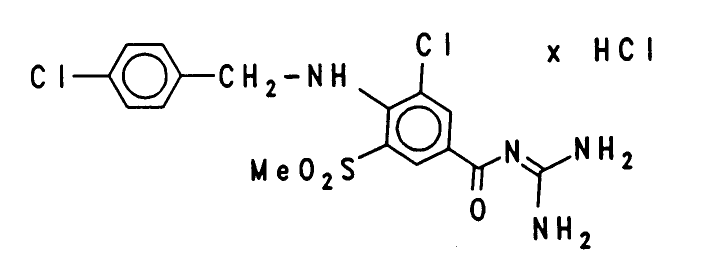

3-Chlor-4-(4-chlorbenzylamino)-5-methylsulfonyl-guandin-hydrochlorid erhält man gemäß Vorschrift aus 3-Chlor-4-(4-chlorbenzylamino)-5-methylsulfonylbenzoesäure und Guanidin. Farblose Kristalle; Schmp. 209-219°C.

### Beispiel 22

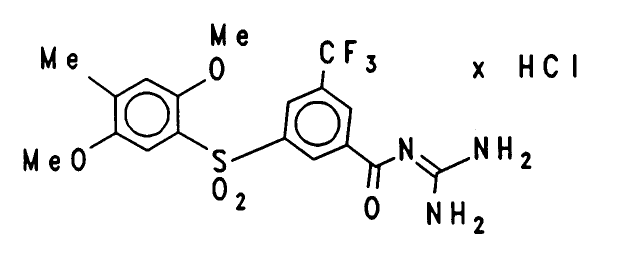

3-(2,5-Dimethoxy-4-methylphenylsulfonyl)-5-trifluormethylbenzoyl-guanidin-hydrochlorid erhält man gemäß Vorschrift aus 3-(2,5-Dimethoxy-4-methylphenylsulfonyl)-5-trifluormethylbenzoesäure und Guanidin.
Farblose Kristalle; Schmp.: 174°C.

### Beispiel 23

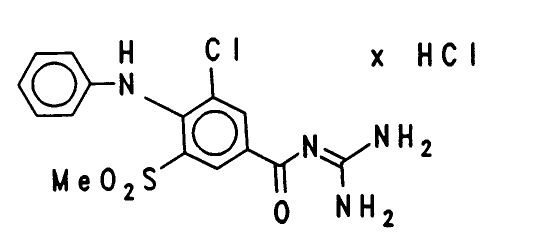

4-Anilino-3-chlor-5-methylsulfonylbenzoyl-guanidin-hydrochlorid erhält man gemäß Vorschrift aus 4-Anilino-3-chlor-5-methylsulfonylbenzoesäure und Guanidin.
Farblose Kristalle; Schmp.: 174°C.
4-Anilino-3-chlor-5-methylsulfonylbenzoesäure (Schmp. 187-189°C) erhält man durch
1. Erhitzen von 3,4-Dichlor-5-methylsulfonylbenzoesäure-methylester in überschüssigem Anilin über 8-10 Stunden auf 120°C und Abtrennung des 4-Anilino-3-chlor-5-methylsulfonylbenzoesäuremethylesters (Schmp. 165-169°C) durch Behandeln der Reaktionsmischung mit 1n HCl und
2. durch nachfolgende Hydrolyse des 4-Anilino-3-chlor-5-methylsulfonylbenzoesäure-methylesters mit methanolisch-wäßriger NaOH und Behandlung des Rückstandes mit 2n HCl nach Abdestillieren des Lösungsmittelgemisches.

## Patentansprüche

1. Benzoylguanidine der Formel I 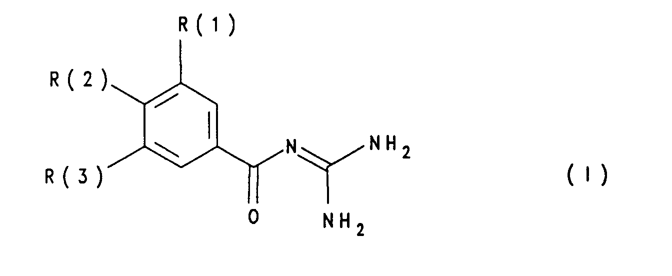 worin bedeuten:
R(1) R(4)-SOₘ oder R(5)R(6)N-SO₂-, mit
m gleich Null, 1 oder 2,
R(4) und R(5) gleich C₁-C₈-Alkyl, C₃-C₆-Alkenyl, -CₙH₂ₙ-R(7),
n gleich Null, 1, 2, 3 oder 4,
R(7) gleich C₅-C₇-Cycloalkyl, Phenyl, welches unsubstituiert ist oder substituiert mit 1-3 Substituenten aus der Gruppe
F, Cl, CF₃, Methyl, Methoxy oder NR(8)R(9) mit
R(8) und R(9) gleich H oder C₁-C₄-Alkyl,
wobei R(5) auch in der Bedeutung von H steht,
R(6) gleich H oder C₁-C₄-Alkyl,
wobei R(5) und R(6) gemeinsam 4 oder 5 Methylengruppen sein können, von denen eine CH₂-Gruppe durch ein O, S, NH, N-CH₃ oder N-Benzyl ersetzt sein kann,
R(2) Wasserstoff, F, Cl, Br, (C₁-C₄)-Alkyl-, O-(CH₂)ₘCₚF₂ₚ₊₁, -X-R(10), mit
m gleich Null oder 1,
p gleich 1, 2 oder 3,
X gleich O, S, NR(11),
R(10) gleich H, C₁-C₆-Alkyl, C₅-C₇-Cycloalkyl, Cyclohexylmethyl, Cyclopentylmethyl, -CₙH₂ₙ-R(12) mit
n gleich Null, 1, 2, 3 oder 4 und
R(12) gleich Phenyl, das unsubstituiert oder substituiert ist mit 1-3 Substituenten aus der Gruppe
F, Cl, CF₃, Methyl, Methoxy oder NR(8)R(9) mit
R(8) und R(9) gleich H oder C₁-C₄-Alkyl,
R(11) Wasserstoff, C₁-C₃-Alkyl,
wobei R(10) und R(11) auch gemeinsam 4 oder 5 Methylengruppen sein können und eine CH₂-Gruppe durch O, S, NH, N-CH₃ oder N-Benzyl ersetzt sein kann,
R(3) wie R(1) definiert, oder C₁-C₆-Alkyl, Nitro, Cyano, Trifluormethyl, F, Cl, Br, J,-X-R(10) mit
X gleich O, S, NR(11),
R(10) gleich H, C₁-C₆-Alkyl, C₅-C₇-Cycloalkyl, Cyclohexylmethyl, Cyclopentylmethyl, -CₙH₂ₙ-R(12) mit
n gleich null bis 4 und
R(12) gleich Phenyl, das unsubstituiert ist oder substituiert mit 1 -3 Substituenten aus der Gruppe
F, Cl, CF₃, Methyl, Methoxy oder NR(8)R(9) mit R(8) und
R(9) gleich H oder C₁-C₄-Alkyl,
R(11) C₁-C₃-Alkyl,
wobei R(10) und R(11) auch gemeinsam 4 oder 5 Methylengruppen sein können und eine CH₂-Gruppe durch O, S, NH, N-CH₃ oder N-Benzyl ersetzt sein kann,
sowie deren pharmazeutisch verträgliche Salze,
wobei jedoch Verbindungen ausgenommen sind, in denen gleichzeitig bedeuten:
R(1) R(4)-SOₘ mit m = Null bis 2 oder R(5)R(6) NSO₂-,
R(2) Halogen, (C₁-C₄)-Alkyl, und
R(3) -NR(10)R(11).

2. Verbindung I nach Anspruch 1, dadurch gekennzeichnet, daß bedeuten:
R(1) R(4)-SOₘ oder R(5)R(6)N-SO₂-, mit
m gleich Null, 1 oder 2,
R(4) gleich Methyl oder -CₙH₂ₙ-R(7),
n gleich Null oder 1,
R(7) gleich Phenyl, welches unsubstituiert ist oder substituiert mit 1-3 Substituenten aus der Gruppe
Cl, CF₃, Methyl oder Methoxy,
R(5) gleich H, C₁-C₆-Alkyl, Allyl, -CₙH₂ₙ-R(7),
n gleich Null, 1,2 oder 3,
R(7) gleich Phenyl, welches unsubstituiert ist oder substituiert mit 1-3 Substituenten aus der Gruppe
F, Cl, CF₃, Methyl, Methoxy oder NR(8)R(9) mit
R(8) und R(9) gleich H oder Methyl,
R(6) gleich H oder Methyl,
wobei R(5) und R(6) gemeinsam 4 oder 5 Methylengruppen sein können, von denen eine CH₂-Gruppe durch ein O, S, N-CH₃ oder N-Benzyl ersetzt sein kann,
R(2) Wasserstoff, O-CH₂CF₃ oder -X-R(10), mit
X gleich O, S, NR(11),
R(10) gleich H, C₁-C₆-Alkyl, -CₙH₂ₙ-R(12) mit
n für X in der Bedeutung von Sauerstoff oder Schwefel gleich Null, und für X in der Bedeutung von NR(12) gleich Null oder 1, und
R(12) gleich Phenyl, das unsubstituiert oder substituiert ist mit 1-3 Substituenten aus der Gruppe
F, Cl, CF₃, Methyl,
R(11) gleich Wasserstoff, (C₁-C₃)-Alkyl,
wobei R(10) und R(11) auch gemeinsam 4 oder 5 Methylengruppen sein können und eine CH₂-Gruppe durch O, S, N-CH₃ oder N-Benzyl ersetzt sein kann,
R(3) Methyl, Nitro, Cyano, Trifluormethyl, F, Cl, -X-R(10) mit
X gleich O, S, NR(11),
R(10) gleich H, C₁-C₆-Alkyl, -CₙH₂ₙ-R(12) mit
n gleich null oder 1,
R(12) gleich Phenyl, das unsubstituiert ist oder substituiert mit 1 -3 Substituenten aus der Gruppe Cl, CF₃, Methyl, Methoxy,
R(11) gleich Wasserstoff oder (C₁-C₃)-Alkyl,
wobei R(10) und R(11) auch gemeinsam 4 oder 5 Methylengruppen sein können,
sowie deren pharmazeutisch verträgliche Salze.

3. Verbindung I nach Anspruch 1, dadurch gekennzeichnet, daß bedeuten
R(1) (R4)-SO₂- oder R(5)R(6)N-SO₂- mit
R(4) gleich Methyl,
R(5) und R(6) gleich Wasserstoff,
R(2) Wasserstoff, -O-CH₂-CF₃ oder -X-R(10) mit
X gleich Sauerstoff, N-R(11),
R(10) gleich H, C₁-C₆-Alkyl, -CₙH₂ₙ-R(12) mit
n = Null oder 1
R(12) gleich Phenyl, das unsubstituiert oder substituiert ist mit einem Substituenten aus der Gruppe F, Cl oder Methyl,
R(11) gleich Wasserstoff oder C₁-C₃-Alkyl ist.
R(3) Methyl, F, Cl,
sowie deren pharmakologisch verträgliche Salze.

4. Verbindungen I nach Anspruch 1, ausgewählt aus der Gruppe 3-Chlor-4-N,N-diethylamino-5-methylsulfonylbenzoyl-guanidin-hydrochlorid, 3,4-Dimethyl-5-sulfamoylbenzoyl-guanidin-hydrochlorid.

5. Verfahren zur Herstellung einer Verbindung I nach Anspruch 1, dadurch gekennzeichnet, daß man
Verbindungen der Formel II 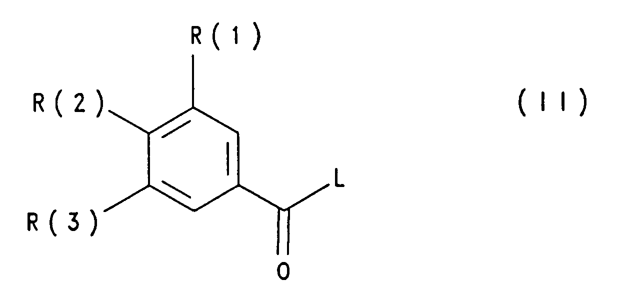 mit Guanidin umsetzt, worin R(1) bis R(3) die angegebene Bedeutung besitzen und L für eine leicht nucleophil substituierbare leaving group steht.

6. Verwendung einer Verbindung I nach Anspruch 1 zur Herstellung eines Medikaments zur Behandlung von Arrhythmien oder der angina pectoris.

7. Verwendung einer Verbindung I nach Anspruch 1 zur Herstellung eines Medikaments zur Behandlung oder Prophylaxe des Herzinfarktes.

8. Verwendung einer Verbindung I nach Anspruch I zur Herstellung eines Medikaments zur Behandlung von ischämischen Zuständen des Herzens.

9. Verwendung einer Verbindung I nach Anspruch I zur Herstellung eines Medikaments zur Behandlung von Krankheiten, bei denen die Proliferation der Fibroblasten eine primäre oder sekundäre Ursache darstellt, und somit ihre Verwendung, zur Herstellung eines Antiatherosklerotikas, Mittel gegen Diabetische Spätkomplikationen, Krebserkrankungen, fibrotische Erkrankungen wie Lungenfibrose, Leberfibrose oder Nierenfibrose.

10. Verwendung einer Verbindung I nach Anspruch 1 zur Herstellung eines wissenschaftliches Tools zur Inhibition des Na+/H+ Exchangers zur Diagnose der Hypertonie und proliferativer Erkrankungen.

## Claims

1. A benzoylguanidine of the formula I 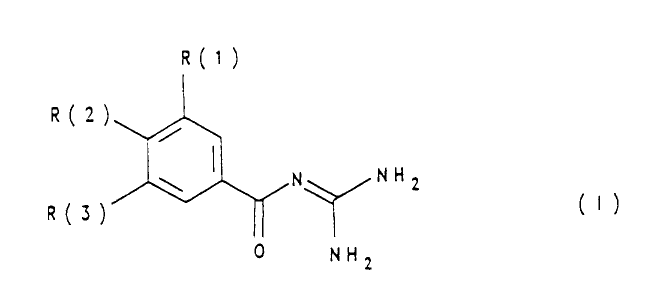 in which:
R(1) is R(4)-SOₘ or R(5)R(6)N-SO₂-, where m is zero, 1 or 2,
R(4) and R(5) are C₁-C₈-alkyl, C₃-C₆-alkenyl or -CₙH₂ₙ-R(7),
n is zero, 1, 2, 3 or 4,
R(7) is C₅-C₇-cycloalkyl or phenyl which is unsubstituted or substituted by 1-3 substituents from the group comprising F, Cl, CF₃, methyl, methoxy and NR(8)R(9) where
R(8) and R(9) are H or C₁-C₄-alkyl,
where R(5) also has the meaning of H,
R(6) is H or C₁-C₄-alkyl,
where R(5) and R(6) can together be 4 or 5 methylene groups, of which one CH₂ group can be replaced by O, S, NH, N-CH₃ or N-benzyl,
R(2) is hydrogen, F, Cl, Br, (C₁-C₄)-alkyl,
O-(CH₂)ₘCₚF₂ₚ₊₁ or -X-R(10), where
m is zero or 1,
p is 1, 2 or 3,
X is O, S, or NR(11),
R(10) is H, C₁-C₆-alkyl, C₅-C₇-cycloalkyl,
cyclohexylmethyl, cyclopentylmethyl or -CₙH₂ₙ-R(12) where
n is zero, 1, 2, 3 or 4 and
R(12) is phenyl which is unsubstituted or substituted by 1-3 substituents from the group comprising
F, Cl, CF₃, methyl, methoxy and NR(8)R(9) where
R(8) and R(9) are H or C₁-C₄-alkyl,
R(11) is hydrogen or C₁-C₃-alkyl,
where R(10) and R(11) can also together be 4 or 5 methylene groups and a CH₂ group can be replaced by O, S, NH, N-CH₃ or N-benzyl,
R(3) is defined as R(1), or is C₁-C₆-alkyl, nitro, cyano, trifluoromethyl, F, Cl, Br, I or -X-R(10) where
X is O, S or NR(11),
R(10) is H, C₁-C₆-alkyl, C₅-C₇-cycloalkyl, cyclohexylmethyl, cyclopentylmethyl or -CₙH₂ₙ-R(12) where
n is from zero to 4 and
R(12) is phenyl which is unsubstituted or substituted by 1-3 substituents from the group comprising
F, Cl, CF₃, methyl, methoxy and NR(8)R(9) where R(8) and R(9) are H or C₁-C₄-alkyl,
R(11) is H or C₁-C₃-alkyl,
where R(10) and R(11) can also together be 4 or 5 methylene groups and a CH₂ group can be replaced by O, S, NH, N-CH₃ or N-benzyl,
and their pharmaceutically tolerable salts,
but where compounds are excluded in which, at the same time:
R(1) is R(4)-SOₘ where m = zero to 2 or R(5)R(6) NSO₂-,
R(2) is halogen, (C₁-C₄)-alkyl, and
R(3) is -NR(10)R(11).

2. A compound I as claimed in claim 1, wherein:
R(1) is R(4)-SOₘ or R(5)R(6)N-SO₂-, where
m is zero, 1 or 2,
R(4) is methyl or -CₙH₂ₙ-R(7),
n is zero or 1,
R(7) is phenyl which is unsubstituted or substituted by 1-3 substituents from the group comprising
Cl, CF₃, methyl and methoxy,
R(5) is H, C₁-C₆-alkyl, allyl or -CₙH₂ₙ-R(7),
n is zero, 1, 2 or 3,
R(7) is phenyl which is unsubstituted or substituted by 1-3 substituents from the group comprising
F, Cl, CF₃, methyl, methoxy and NR(8)R(9) where R(8) and R(9) are H or methyl,
R(6) is H or methyl,
where R(5) and R(6) can together be 4 or 5 methylene groups, of which one CH₂ group can be replaced by O, S, N-CH₃ or N-benzyl,
R(2) is hydrogen, O-CH₂CF₃ or -X-R(10), where X is O, S or NR(11),
R(10) is H, C₁-C₆-alkyl or -CₙH₂ₙ-R(12) where n for X having the meaning of oxygen or sulfur is zero, and for X having the meaning of NR(12) is zero or 1, and
R(12) is phenyl which is unsubstituted or substituted by 1-3 substituents from the group comprising
F, Cl, CF₃ and methyl,
R(11) is hydrogen or (C₁-C₃)-alkyl,
where R(10) and R(11) can also together be 4 or 5 methylene groups and a CH₂ group can be replaced by O, S, N-CH₃ or N-benzyl,
R(3) is methyl, nitro, cyano, trifluoromethyl, F, Cl or -X-R(10) where
X is O, S or NR(11),
R(10) is H, C₁-C₆-alkyl or -Cₙ-H₂ₙ-R(12) where n is zero or 1,
R(12) is phenyl which is unsubstituted or substituted by 1-3 substituents from the group comprising Cl, CF₃, methyl and methoxy,
R(11) is hydrogen or (C₁-C₃)-alkyl,
where R(10) and R(11) can also together be 4 or 5 methylene groups, and their pharmaceutically tolerable salts.

3. A compound I as claimed in claim 1, wherein:
R(1) is R(4)-SO₂- or R(5)R(6)N-SO₂- where
R(4) is methyl,
R(5) and R(6) are hydrogen,
R(2) is hydrogen, -O-CH₂-CF₃ or X-R(10) where X is oxygen or N-R(11),
R(10) is H, C₁-C₆-alkyl or -CₙH₂ₙ-R(12) where
n = zero or 1
R(12) is phenyl which is unsubstituted or substituted by a substituent from the group comprising F, Cl and methyl,
R(11) is hydrogen or C₁-C₃-alkyl,
R(3) is methyl, F or Cl, and their pharmacologically tolerable salts.

4. A compound I as claimed in claim 1, selected from the group comprising 3-chloro-4-N,N-diethylamino-5-methylsulfonylbenzoylguanidine hydrochloride and 3,4-dimethyl-5-sulfamoylbenzoylguanidine hydrochloride.

5. A process for the preparation of a compound I as claimed in claim 1, which comprises
reacting a compound of the formula II 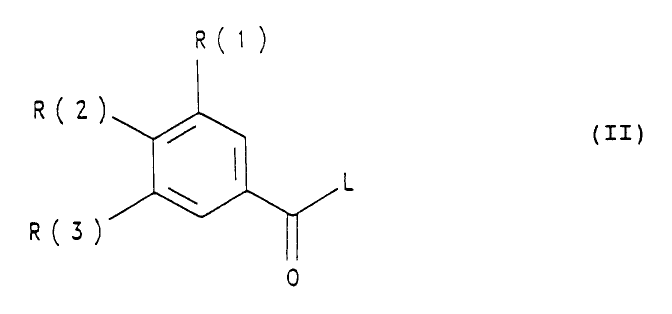 with guanidine, in which R(1) to R(3) have the given meaning and L is a leaving group which can be easily nucleophilically substituted.

6. The use of a compound I as claimed in claim 1 for the production of a medicament for the treatment of arrhythmias or angina pectoris.

7. The use of a compound I as claimed in claim 1 for the production of a medicament for the treatment or prophylaxis of cardiac infarct.

8. The use of a compound I as claimed in claim 1 for the production of a medicament for the treatment of ischemic conditions of the heart.

9. The use of a compound I as claimed in claim 1 for the production of a medicament for the treatment of diseases in which the proliferation of the fibroblasts is a primary or secondary cause, and thus their use for the production of an anti-atherosclerotic or agent against late-onset diabetic complications, cancers and fibrotic diseases such as pulmonary fibrosis, fibrosis of the liver or fibrosis of the kidneys.

10. The use of a compound I as claimed in claim 1 to produce a scientific tool to inhibit the Na⁺/H⁺ exchanger to diagnose hypertension and proliferative diseases.

## Revendications

1. Benzoylguanidines de formule I 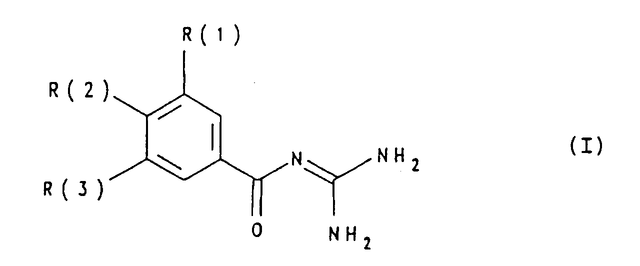 dans laquelle
R(1) représente un groupe R(4)-SOₘ ou R(5)R(6)N-SO₂-,
m étant égal à zéro, 1 ou 2,
R(4) et R(5) représentant un groupe alkyle en C₁-C₈,
alcényle en C₃-C₆, -CₙH₂ₙ-R(7),
n étant égal à zéro, 1, 2, 3 ou 4,
R(7) représentant un groupe cycloalkyle en C₅-C₇,
un groupe phényle qui est non substitué ou substitué par 1-3 substituants choisis parmi F, Cl, CF₃, un groupe méthyle, méthoxy ou NR(8)R(9) dans lequel R(8) et R(9) représentent H ou un groupe alkyle en C₁-C₄,
R(5) représentant également H,
R(6) représentant H ou un groupe alkyle en C₁-C₄,
R(5) et R(6) pouvant être ensemble 4 ou 5 groupes
méthylène, dont un groupe CH₂ peut être remplacé
par O, S ou par un groupe NH, N-CH₃ ou N-benzyle,
R(2) représente un atome d'hydrogène ou de F, Cl, Br, ou un groupe alkyle en C₁-C₄, O-(CH₂)ₘCₚF₂ₚ₊₁, -X-R(10),
m étant égal à zéro ou 1,
p étant égal à 1, 2 ou 3,
X représentant O, S, NR(11),
R(10) représentant H ou un groupe alkyle en C₁-C₆,
cycloalkyle en C₅-C₇, cyclohexylméthyle, cyclopentylméthyle, -CₙH₂ₙ-R(12) dans lequel
n est égal à zéro, 1, 2, 3 ou 4 et
R(12) représente un groupe phényle qui est non substitué ou substitué par 1-3 substituants choisis parmi
F, Cl, CF₃, un groupe méthyle, méthoxy ou NR(8)R(9), R(8) et R(9) représentant H ou un groupe alkyle en C₁-C₄,
R(11) représente un atome d'hydrogène ou un groupe alkyle en C₁-C₃,
R(10) et R(11) pouvant également être ensemble 4 ou 5 groupes méthylène et un groupe CH₂ pouvant être remplacé par O, S ou par un groupe NH, N-CH₃ ou N-benzyle,
R(3) est défini comme R(1) ou représente un groupe alkyle en C₁-C₆, nitro, cyano, trifluorométhyle, F, Cl, Br, I, ou un groupe -X-R(10) dans lequel
X représente O, S, NR(11),
R(10) représente H ou un groupe alkyle en C₁-C₆, cycloalkyle en C₅-C₇, cyclohexylméthyle, cyclopentylméthyle, -CₙH₂ₙ-R(12) dans lequel
n va de zéro à 4 et
R(12) représente un groupe phényle qui est non substitué ou substitué par 1-3 substituants choisis parmi
F, Cl, CF₃, un groupe méthyle, méthoxy ou NR(8)R(9) dans lequel R(8) et R(9) représentent H ou un groupe alkyle en C₁-C₄,
R(11) représente un groupe alkyle en C₁-C₃,
R(10) et R(11) pouvant également être ensemble 4 ou 5 groupes méthylène, et un groupe CH₂ pouvant être remplacé par O, S ou par un groupe NH, N-CH₃ ou N-benzyle,
et ses sels pharmaceutiquement acceptables, les composés dans lesquels simultanément
R(1) représente un groupe R(4)-SOₘ dans lequel m va de zéro à 2, ou R(5)R(6)N-SO₂-,
R(2) représente un atome d'halogène ou un groupe alkyle en C₁-C₄ et
R(3) représente un groupe -NR(10)R(11) étant toutefois exclus.

2. Composé I selon la revendication 1, caractérisé en ce que
R(1) représente un groupe R(4)-SOₘ ou R(5)R(6)N-SO₂-,
m étant égal à zéro, 1 ou 2,
R(4) représentant un groupe méthyle ou -CₙH₂ₙ-R(7),
n étant égal à zéro ou 1,
R(7) représentant un groupe phényle qui est non substitué ou substitué par 1-3 substituants choisis parmi
Cl, CF₃, le groupe méthyle ou méthoxy,
R(5) représentant H ou un groupe alkyle en C₁-C₆,
allyle, -CₙH₂ₙ-R(7),
n étant égal à zéro, 1, 2 ou 3,
R(7) représentant un groupe phényle qui est non substitué ou substitué par 1-3 substituants choisis parmi F, Cl, CF₃, un groupe méthyle, méthoxy ou NR(8)R(9) dans lequel R(8) et R(9) représentent H ou le groupe méthyle,
R(6) représentant H ou le groupe méthyle,
R(5) et R(6) pouvant être ensemble 4 ou 5 groupes méthylène, dont un groupe CH₂ peut être remplacé par O, S ou par le groupe N-CH₃ ou N-benzyle,
R(2) représente un atome d'hydrogène ou un groupe O-CH₂CF₃ ou -X-R(10) dans lequel
X représente O, S, NR(11),
R(10) représente H ou un groupe alkyle en C₁-C₆, -CₙH₂ₙ-R(12),
n est égal à zéro lorsque X représente un atome d'oxygène ou de soufre, et est égal à zéro ou 1 lorsque X représente NR(11), et
R(12) représentant un groupe phényle qui est non substitué ou substitué par 1-3 substituants choisis parmi F, Cl, CF₃, le groupe méthyle,
R(11) représente un atome d'hydrogène ou un groupe alkyle en C₁-C₃,
R(10) et R(11) pouvant également être ensemble 4 ou 5 groupes méthylène et un groupe CH₂ pouvant être remplacé par O, S ou par un groupe N-CH₃ ou N-benzyle,
R(3) représente le groupe méthyle, nitro, cyano, trifluorométhyle, F, Cl ou un groupe -X-R(10) dans lequel
X représente O, S, NR(11),
R(10) représente H ou un groupe alkyle en C₁-C₆ ou -CₙH₂ₙ-R(12),
n étant égal à zéro ou 1,
R(12) représentant un groupe phényle qui est non substitué ou substitué par 1-3 substituants choisis parmi Cl, CF₃, le groupe méthyle, méthoxy,
R(11) représentant un atome d'hydrogène ou un groupe alkyle en C₁-C₃,
R(10) et R(11) pouvant également être ensemble 4 ou 5 groupes méthylène,
et ses sels pharmaceutiquement acceptables.

3. Composé I selon la revendication 1, caractérisé en ce que
R(1) représente R(4)-SO₂- ou R(5)R(6)N-SO₂-,
R(4) représentant le groupe méthyle,
R(5) et R(6) représentant un atome d'hydrogène,
R(2) représente un atome d'hydrogène, un groupe -O-CH₂-CF₃ ou X-R(10) dans lequel
X représente un atome d'oxygène ou le groupe N-R(11),
R(10) représente H, un groupe alkyle en C₁-C₆,
-CₙH₂ₙ-R(12) dans lequel
n est égal à zéro ou 1,
R(12) représente le groupe phényle qui est non substitué ou substitué par un substituant choisi parmi F, Cl et le groupe méthyle,
R(11) représente un atome d'hydrogène ou un groupe alkyle en C₁-C₃,
R(3) représente F, Cl ou le groupe méthyle,
et ses sels pharmacologiquement acceptables.

4. Composés I selon la revendication 1, choisis parmi les suivants:
3-chloro-4-N,N-diéthylamino-5-méthylsulfonylbenzoylguanidine chlorhydrate,
3,4-diméthyl-5-sulfamoylbenzoylguanidine chlorhydrate.

5. Procédé pour la préparation d'un composé I selon la revendication 1, caractérisé en ce que l'on fait réagir avec de la guanidine des composés de formule II 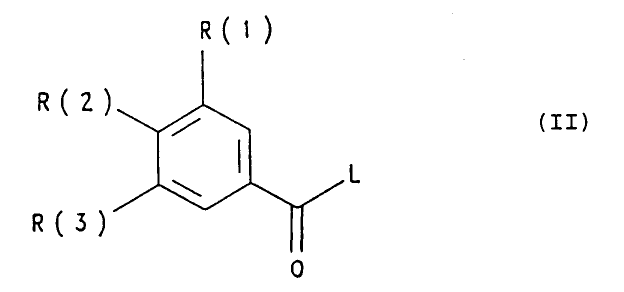 dans laquelle R(1) à R(3) ont les significations données et L représente un groupe partant aisément remplaçable par substitution nucléophile.

6. Utilisation d'un composé I selon la revendication 1, pour la fabrication d'un médicament destiné au traitement d'arythmies ou de l'angine de poitrine.

7. Utilisation d'un composé I selon la revendication 1, pour la fabrication d'un médicament destiné au traitement ou la prophylaxie de l'infarctus du myocarde.

8. Utilisation d'un composé I selon la revendication 1, pour la fabrication d'un médicament destiné au traitement d'états ischémiques du coeur.

9. Utilisation d'un composé I selon la revendication 1, pour la fabrication d'un médicament destiné au traitement de maladies dans lesquelles la prolifération des fibroblastes représente une cause primaire ou secondaire, et ainsi leur utilisation pour la fabrication d'un médicament anti-athérosclérose, de médicaments contre des complications diabétiques tardives, des maladies cancéreuses, des maladies fibreuses telles que la fibrose pulmonaire, la fibrose hépatique ou la fibrose rénale.

10. Utilisation d'un composé I selon la revendication 1, pour la fabrication d'un outil scientifique pour l'inhibition de l'échangeur Na+/H+, dans le diagnostic de l'hypertonie et de maladies prolifératives.
